# EUROPEAN PATENT APPLICATION

(11) **EP 1 889 611 A1**
(43) Date of publication of application: **20.02.2008**
(21) Application number: 06766530.7
(22) Date of filing: 09.06.2006
(51) Int. Cl.: A61K 9/36, A61K 9/16, A61K 9/20, A61K 31/4035, A61K 47/10, A61K 47/26, A61P 25/16, A61P 25/28

(54) **SUGAR-COATED TABLET**

(30) Priority: 10.06.2005 JP 2005171443
(71) Applicant: Takeda Pharmaceutical Company Limited, Osaka-shi, Osaka 541-0045 (JP)
(72) Inventor: UCHIYAMA, Yoshihiro, TAKEDA PHARMACEUTICAL CO. LTD, Osaka-shi, Osaka 5328686 (JP); NAKANO, Yoshinori, TAKEDA PHARMACEUTICAL CO. LTD., Osaka-shi, Osaka 5328686 (JP)
(74) Representative: Dossmann, Gérard
(86) International application number: PCT/JP2006/311618
(87) International publication number: WO 2006/132360

(57) **Abstract**

A preparation wherein an active ingredient unstable to oxygen is stabilized by coating a portion containing the active ingredient unstable to oxygen with a sugar-coated layer containing (1) sugar alcohol as a sugar-coating base material and (2) a binder is provided.

## Description

### Technical Field

The present invention relates to a sugar-coated preparation.

### Background Art

Of pharmaceutically effective compounds, there is a compound unstable to oxygen (that is, a compound which is easy to be oxidized). Since it is difficult to provide a stable preparation containing such a compound as an active ingredient by conventional production technologies, the compound has been often excluded from pharmaceutical candidate compounds in first place. In case of developing a preparation of the compound, some devices for the preparation such as storing it in a container made of materials having low oxygen permeability, additionally flushing the space inside the container with the gas other than oxygen, alternatively enclosing an oxygen absorbent or an oxygen remover in the container for the purpose of removing oxygen in the container are required. As a technology to solve such a problem, the stabilized film coated tablet containing, as an active ingredient, a nitrogen-containing condensed heterocyclic compound unstable to oxygen is described in WO 03-051355. In addition, substances which are more unstable than the active ingredient unstable to oxygen (for example, an orally administrable antioxidant) are incorporated into the preparation to prevent the compound from being oxidized.

Among these devices for the preparation, the device using gas flushing and the oxygen absorbent or remover causes limitations for use at the medical site since it is difficult to ensure stability after having opened the container for medication administration; for example, the preparation is required to store in low temperature places such as refrigerators after opening so that oxidation of the preparation is delayed. Besides, due to limitation in the number of the preparation filled into the container, larger storage places for the preparations are required. Thus, they are inferior in user-friendly aspects. Meanwhile, incorporation of substances, which are more unstable than the active ingredient unstable to oxygen, into the preparation contributes to stabilization in a preparation manufacturing process. If effects of the stabilization are remarkable, it is not required to have the above special packaging forms, but the incorporation of the substances into the preparation depends on physicochemical properties of the target compound. Further, there are not many orally administrable antioxidants from a safety standpoint. For example, because it is limited to BHT, BHA, an ascorbic acid, etc. in Europe, the probability to design the pharmaceutical devices is not always high.

Under these circumstances, the diversification of the preparation in which an active ingredient unstable to oxygen is stabilized and the development of more excellent preparations are desired since it can be expected that the pharmaceutical candidate compounds are widely selected and thus superior drugs can be provided if the preparation wherein such active ingredient is stabilized could be provided.
Patent Application 1: WO 03-051355

### Disclosure of the Invention

### Problems to be solved by the Invention

It is an object of the present invention to provide a preparation wherein the oxidation of an active ingredient unstable to oxygen is suppressed.

### Means for Solving the Problems

The present inventors have found that the oxidation of an active ingredient unstable to oxygen can be suppressed by incorporating a binder into a sugar-coated layer, and thereby suppressing the oxygen permeation. As a result of further investigations based on this finding, the present inventors have completed the present invention.

That is, the present invention provides:
[1] A preparation which comprises a portion containing an active ingredient unstable to oxygen and a sugar-coated layer containing (1) sugar alcohol as a sugar-coating base material and (2) a binder, wherein the portion is coated with the sugar-coated layer;
[2] The preparation as described in the above [1], wherein the sugar alcohol is one or more sugar alcohols selected from erythritol, mannitol, xylitol and sorbitol;
[3] The preparation as described in the above [1], wherein the sugar alcohol is erythritol;
[4] The preparation as described in the above [1], which further contains a sugar as the sugar-coating base material;
[5] The preparation as described in the above [1], wherein the binder is gum arabic;
[6] The preparation as described in the above [1] which further comprises a film coat layer, wherein the sugar-coated layer is coated with the film coat layer;
[7] The preparation as described in the above [1], which further comprises an antioxidant;
[8] The preparation as described in the above [1], wherein the active ingredient unstable to oxygen is (R)-5,6-dimethoxy-2-[2,2,4,6,7-pentamethyl-3-(4-methylphenyl)-2,3-dihydro-1-benzofuran-5-yl]isoindoline;
[9] The preparation as described in the above [1], wherein the weight of the sugar-coated layer is about 20% to about 40% of the total weight of the preparation;
[10] The preparation as described in the above [1], wherein the sugar-coated layer contains the binder of about 1 to about 50% (w/w);
[11] The preparation as described in the above [1], which is a tablet;
[12] A method for suppressing the oxidation of an active ingredient, which comprises, in a preparation containing an active ingredient oxidizable by oxygen, coating a portion containing the active ingredient with a sugar-coated layer containing (1) sugar and/or sugar alcohol as a sugar-coating base material and (2) a binder;
[13] A method for suppressing oxygen permeation of a sugar-coated layer, which comprises, in a preparation which comprises a portion containing an active ingredient oxidizable by oxygen and a sugar-coated layer, incorporating a binder into the sugar-coated layer; and
[14] A method for producing a sugar-coated preparation wherein the oxidation of an active ingredient is suppressed, which comprises coating a portion containing the active ingredient oxidizable by oxygen with a sugar-coated layer containing (1) sugar and/or sugar alcohol as a sugar-coating base material and (2) a binder.

### Effect of the Invention

According to this invention, a preparation wherein the oxidation of an active ingredient unstable to oxygen is suppressed can be provided.

### Brief Description of Drawings

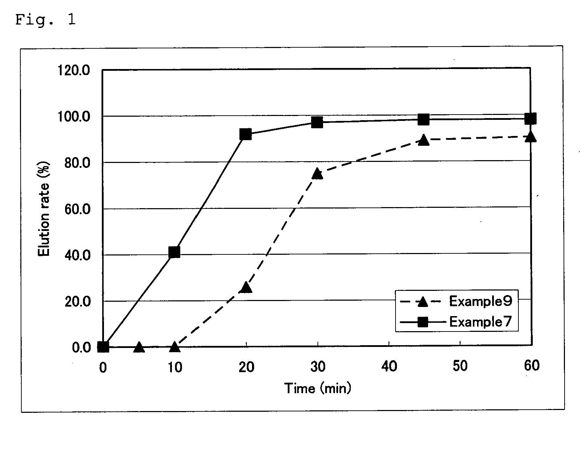

Fig. 1 is a graph showing elution profile.

### Best Mode for Carrying Out the Invention

The present invention can be applied to an active ingredient oxidizable by oxygen, and in particular can be suitably applied to an active ingredient unstable to oxygen since the preparation of the present invention has particularly high oxidation suppressing effect. The term "unstable to oxygen", as used herein, means that it is more easily oxidized than sodium ascorbate under some conditions.

More specifically, an active ingredient in which a residual ratio measured after leaving it at 40°C and 75% RH in the air for 1 month is lower than a residual ratio of sodium ascorbate as a comparative control, is an active ingredient unstable to oxygen. Specific residual ratio of the "active ingredient unstable to oxygen" under some conditions used in the invention is preferably 98% (w/w) or less, more preferably 95% (w/w) or less, and even more preferably 92% (w/w) or less. Of active ingredients unstable to oxygen, a nitrogen-containing heterocyclic compound unstable to oxygen (e.g., an isoindoline compound and a dihydropyridine compound) is preferable.

Examples of such a compound include (R)-5,6-dimethoxy-2-[2,2,4,6,7-pentamethyl-3-(4-methylphenyl)-2,3-dihydro-1-benzofuran-5-yl]isoindoline (hereinafter sometimes referred to as Compound A). Compound A is a known compound described in WO 00-34262 and can be produced by a method described therein. Here, the active ingredient oxidizable by oxygen may be used in one kind or in a combination of two or more kinds.

Hereinafter, the present invention is explained further in details with reference to a case where the "active ingredient unstable to oxygen" is used as the active ingredient, but the same also applies to a case where the "active ingredient oxidizable by oxygen" is used as the active ingredient, as readily understood by a person skilled in the art.

The preparation of the present invention has a "portion containing an active ingredient unstable to oxygen". The "portion" is coated with the sugar-coated layer to be described in detail below. Here, the "portion" has only to be present in the inside of the sugar-coated layer and the form thereof is not limited. Further, it is not particularly limited regarding how to be present the active ingredient unstable to oxygen in the "portion".

The content of "the active ingredient unstable to oxygen" in the "portion" is not particularly limited, but usually about 0.1% (w/w) to about 95% (w/w), preferably about 1.0% (w/w) to about 80% (w/w), and more preferably about 1.5% (w/w) to about 70% (w/w).

As apparent from the above description, a preparation which has an intermediate layer isolating the active ingredient from the sugar-coated layer, is also within the scope of the present invention. In this case, the intermediate layer constitutes the "portion containing an active ingredient unstable to oxygen". Examples of the intermediate layer include a waterproof film, an enteric-coated film, a sustained-release film and an anchor coat for preventing from being contacted with the sugar-coated layer, but are not limited to these. More specifically, the intermediate layer may be not a film of a polymer-like substance, but, for example, may be a layer containing edible substances, e.g., at least one excipient used in an orally-administered preparation and may also contain a binder, etc. Although it is obvious, the edible substances may be contained in the waterproof film, the enteric-coated film, the sustained-release film and the anchor coat for preventing from being contacted with the sugar-coated layer described above. In addition, the intermediate layer does not have to be used in one kind and the preparation of the present invention may comprise a plurality of the intermediate layers.

Examples of the form of the "portion containing an active ingredient unstable to oxygen" include a tablet obtained by tableting powders containing the active ingredient unstable to oxygen. Here, as apparent from the above description, the active ingredient does not have to be homogeneously present and may be localized in the inside portion of the sugar-coated layer. As such, examples of the "inside portion" in which the active ingredient is localized, in case where a tablet is used as the inside portion of the sugar-coated layer, include (1) tablets obtained by tableting a mixture of powders containing the above-mentioned active ingredient and powders consisting of substances other than the above-mentioned active ingredient, (2) tablets called the so-called laminated tablets in which the active ingredient is contained in at least one of plural layers, and (3) tablets called the so-called dry-coated tablets in which any one of an inner core and an outer core contains the active ingredient.

Further, examples of the form of the portion containing the active ingredient in the present invention include granules, in addition to tablets. Examples of the granules include a form in which the core made of an edible substance is coated with the excipient or the active ingredient, but are not limited to this. The core does not have to be homogeneously coated and may be coated in a multilayered structure. Further, the active ingredient may be heterogeneously or homogeneously contained in at least one layer of them.

Furthermore, other examples of the form of the portion containing the active ingredient in the present invention include the so called capsules in which at least the active ingredient is contained in capsules made of edible substances, as typified by gelatin capsules, HPMC capsules and pullulan capsules.

Further in the present invention, as described above, the active ingredient unstable to oxygen does not have to be used in one kind and can be also used in combination with one or more other active ingredients which do not belong to the aforementioned active ingredient (i.e., active ingredients other than the active ingredient unstable to oxygen). In this case, the active ingredient which does not belong to the aforementioned active ingredient may be incorporated into the same portion as that in which the active ingredient is contained, or may be incorporated into portions other than the same portion. For example, in the aforementioned laminated tablets, the active ingredient which does not belong to the aforementioned active ingredient may be incorporated into a layer in which the active ingredient is contained, or may be incorporated into other layers. The same applies to other embodiments other than the laminated tablets.

The inside portion of the sugar-coated layer may contain pharmaceutically acceptable additives.

Examples of the pharmaceutically acceptable additives include excipients, disintegrants, antioxidants, fluidizers, binders, lubricants, coloring agents and flavoring agents. The antioxidant may be contained in the preparation of the present invention from the viewpoint of oxidation prevention during the production thereof. These additives may be used alone or in combination of two or more kinds thereof. For the purpose of improving productivity, granulated mannitol and silicic anhydride may be also contained in the preparation. Thus, as a result of improving productivity, miniaturization of the preparation and reduction in production time are also obtained.

Examples of the excipients include lactose, white sugar, mannitol, starch, cornstarch, microcrystalline cellulose and light anhydrous silicic acid.

Examples of the disintegrants include starch, carboxymethylcellulose, carboxymethylcellulose calcium, croscarmellose sodium, carboxymethyl starch sodium and hydroxypropylcellulose.

Examples of the antioxidants include ascorbic acid or a salt thereof (e.g., a sodium salt, a calcium salt, a magnesium salt, a potassium salt, a salt of an inorganic basic substance, a basic amino acid salt, a meglumine salt and the like), sodium nitrite, L-ascorbic acid stearic acid ester, sodium hydrogen sulfite, sodium sulfite, a salt of edetic acid (e.g. a sodium salt, a potassium salt, and a calcium salt), erythorbic acid, cysteine hydrochloride, citric acid, tocopherol acetate, cysteine, potassium dichloroisocyanurate, dibutylhydroxytoluene (BHT), soybean lecithin, sodium thioglycolate, thioglycerol, tocopherol (Vitamin E), d-δ-tocopherol, sodium formaldehyde sulfoxylate, ascorbic palmitate, sodium pyrosulfite, butylhydroxyanisole (BHA), 1,3-butylene glycol, benzotriazole, pentaerythrityl tetrakis[3-(3,5-di-tert-butyl-4-hydroxyphenyl)propionate], propyl gallate, and 2-mercaptobenzimidazole. Among these, sodium ascorbate, ascorbic acid, potassium ascorbate, magnesium ascorbate and calcium ascorbate are preferred, and the antioxidants may be used in combination as a mixture of a plurality of them. The antioxidants may be incorporated in the state in which they are homogeneously mixed with the active ingredient, but may be heterogeneously mixed therewith. Further, the antioxidants may be incorporated into a portion different from the portion in which the active ingredient is contained. For example, in the form of the laminated tablet, the antioxidants may be incorporated into a layer different from the layer in which the active ingredient is contained, the intermediate layer or the sugar-coated layer. The antioxidants may be incorporated into any portion of the preparation provided by the present invention.

Examples of the fluidizers include light anhydrous silicic acid, calcium silicate and aluminum silicate.

Examples of the lubricants include magnesium stearate, calcium stearate, talc and colloidal silica.

Examples of the binders include microcrystalline cellulose, white sugar, mannitol, dextrin, hydroxypropylcellulose, hydroxypropylmethylcellose, polyvinylpyrrolidone, starch, sucrose, gelatin, methylcellulose, carboxymethylcellulose sodium and pullulan.

The preparation of the present invention has the sugar-coated layer. Here, the "sugar-coated" means not limited to one using only sugar as the sugar-coating base material, and sugar and/or sugar alcohol are (is) used as the sugar-coating base material of the sugar-coated layer. The content of the "sugar-coating base material" in the "sugar-coated layer" is not particularly limited, but usually about 10% (w/w) to about 99% (w/w), preferably about 25% (w/w) to about 90% (w/w).

The weight of the sugar-coated layer in the preparation of the present invention is lower than that of the sugar-coated layer in the general preparation and varies depending upon forms of the preparation. For example, in the case of the tablet, the weight is preferably about 20% to about 50%, more preferably about 20% to about 40% of the total weight of the preparation.

Examples of the sugar include sucrose and trehalose. Examples of the sugar alcohol include erythritol, mannitol, xylitol and sorbitol.

As the sugar-coating base material, sugar alcohol is preferable and erythritol is particularly preferable in terms of suppressing the oxidation of an active ingredient, improving productivity, thinning of the sugar-coated layer and leachability of a water-insoluble active ingredient. Here, the sugar-coating base material may be used by combining sugar alcohol with sugar.

The sugar-coated layer constituting the preparation of the present invention contains the binder. The content of the "binder" in the "sugar-coating base material" is not particularly limited, but usually about 1% (w/w) to about 50% (w/w), preferably about 5% (w/w) to about 15% (w/w).

Examples of the binder include gum arabic, pullulan, hydroxypropylcellulose (HPC), hydroxypropylmethylcellulose 2208 (HPMC 2208), hydroxypropylmethylcellulose 2906 (HPMC 2906), hydroxypropylmethylcellulose 2910 (HPMC 2910), methylcellulose (MC), hydroxyethylcellulose (HEC), microcrystalline cellulose, powdered cellulose, low-substituted hydroxypropylcellulose, dextrin, corn starch, pregelatinized starch, partially pregelatinized starch, hydroxypropyl starch, polyvinylpyrrolidone (PVP), carboxy vinyl polymer, polyvinyl alcohol (PVA), microcrystalline cellulose-carmellose sodium, gelatin, xanthan gum, gum tragacanth, powdered tragacanth, macrogol 200, macrogol 300, macrogol 400, macrogol 600, macrogol 1000, macrogol 1500, macrogol 1540, macrogol 4000, macrogol 6000, macrogol 20000 and polyoxyethylene [105] polyoxypropylene [5] glycol.

As the binder, gum arabic is particularly preferred.

The sugar-coated layer containing erythritol as the sugar-coating base material and gum arabic as the binder is particularly preferred.

The sugar-coated layer of the preparation of the present invention may contain pharmaceutically acceptable additives, in addition to the sugar-coating base material and the binder. Examples of the additives include a masking agent, a potentiator, a fluidizer, a coloring agent and a flavoring agent. Examples of the masking agent include titanium dioxide, talc, calcium carbonate, magnesium carbonate and barium sulfate. Examples of the potentiator include microcrystalline cellulose. Examples of the fluidizer include talc.

Examples of the "coloring agent" include yellow ferric oxide, ferric oxide, titanium dioxide and riboflavin.

The "flavoring agent" may be any of synthetic materials and natural products and examples thereof include a lemon flavor, a lime flavor, an orange flavor, a strawberry flavor and menthol.

The preparation of the present invention further may comprise a film coat layer coating the sugar-coated layer, if necessary. The film coat layer may be directly coated on the sugar-coated layer, or an intermediate layer or the like may be present between the sugar-coated layer and the film coat layer.

Examples of the film base material of the film coat layer include hydroxypropylmethylcellulose and hydroxypropylcellulose.

The film coat layer may contain pharmaceutically acceptable additives, in addition to the film coat base material. Examples of the additives include a plasticizer, a masking agent, a fluidizer and a coloring agent.

Examples of the plasticizer include polyethylene glycol.

Examples of the masking agent include titanium dioxide, talc, calcium carbonate, magnesium carbonate and barium sulfate.

Examples of the fluidizer include talc.

Examples of the coloring agent include yellow ferric oxide, ferric oxide, titanium dioxide and riboflavin.

Further, the form of the preparation of the present invention is not particularly limited as long as the portion containing the active ingredient unstable to oxygen is coated with the sugar-coated layer containing sugar or sugar alcohol as the sugar-coating base material and the binder, but is usually a tablet or granule, preferably a tablet.

The preparation of the present invention can be prepared by a conventional manner depending on the form of the preparation.

In the case of a tablet in which a plain tablet containing the active ingredient unstable to oxygen is coated with the sugar-coated layer, for example, the tablet can be prepared as described below, according to a conventional manner.

The active ingredient unstable to oxygen is mixed with a suitable excipient and a binder to be added, if necessary and the resulting mixture is subjected to granulation and then tableting to give tablets. Further, if necessary, according to a conventional manner, the plain tablet may be coated with a waterproof film. The sugar-coating base material, the binder, and the masking agent and the potentiator to be added, if necessary, are dissolved or suspended in purified water in an appropriate proportion to prepare a sugar coating solution. Then, the plain tablet is subjected to sugar-coating by spraying with the sugar coating solution through hands and/or spraying with liquid droplets of 0.1 to 1000 µm.

Furthermore, if necessary, when a film coating is conducted, the aforementioned film coat base material, and additives to be added, if necessary, are dissolved or suspended in purified water in an appropriate proportion to prepare a film coating solution. Then, the surface of the sugar-coated layer is sprayed with the film coating solution using a commercially available coating machine.

Further, as readily understood by a person skilled in the art, the method of the present invention is carried out by containing the binder in the sugar-coated layer of the preparation.

The preparation of the present invention thus obtained can be administered to a subject in the conventional manner.

Further, as described above, the weight of the sugar-coated layer in the preparation of the present invention can be lower than that of the sugar-coated layer in the general preparation since the preparation of the present invention can stabilize highly the active ingredient unstable to oxygen.

For example, examples of a preferred embodiment in the form of the preparation include a tablet which comprises a portion containing an active ingredient unstable to oxygen and a sugar-coated layer containing (1) sugar alcohol as a sugar-coating base material and (2) a binder, wherein the portion is coated with the sugar-coated layer, the weight of the sugar-coated layer is about 20% to about 40% of the total weight of the preparation, and a residual ratio of the active ingredient measured when each preparation is stored in an oxygen gas-filled container (the concentration of oxygen gas of 95% or more) at 40°C for 4 weeks, is about 93% or more (and preferably about 95% or more).

### [EXAMPLES]

Hereinafter, the present invention will be described in more detail with reference to Examples or the like, but they are not intended to limit the present invention.

Additionally, a storage condition in the following Evaluation Examples means storage in the air, unless otherwise noted in the description such as "oxygen gas concentration of 95% or more". Further, in a storage condition of the following Evaluation Examples, no humidity adjusting was carried out unless otherwise described about the humidity adjusting.

### Reference Example 1

A bulk powder of Compound A and sodium ascorbate were respectively left to stand for 1 month at 40°C and 75% RH in the air, and then residual ratios thereof were measured. As a result, the residual ratio of Compound A was 89.7% (W/W), and the residual ratio of sodium ascorbate was 99.0% (W/W).

Quantification of Compound A was carried out by a HPLC method under the following conditions.
Solvent: acetonitrile
Measurement wavelength: 287 nm
Column: CHIRALCEL OJ-R 4.6 mm × 150 mm (manufactured by Daicel Chemical Industries, Ltd.)
Mobile phase: mixed solution of acetonitrile/10 mM aqueous ammonium acetate solution (16 : 9)
Oven temperature: around 25°C
Quantification of sodium ascorbate was carried out by an iodine titration technique (solvent: metaphosphoric acid solution (1→50), indicator: starch test solution).

### Control Example 1

A preparation was prepared according to the formulation shown in Table 1. That is, for a 100 mg tablet, Compound A (19700 g), D-mannitol (34480 g), sodium ascorbate (1970 g) and croscarmellose sodium (Ac-Di-Sol) (2955 g) were put in a fluidized bed granulator (WSG-60, Powrex Corporation), preheated and mixed. A 5% aqueous solution of hydroxypropylcellulose (HPC-L) (39400 g) was sprayed to prepare a granule of Compound A. The resulting granule of Compound A (58590 g) was sized by using a power mill (manufactured by Showa Kagaku Kikaikosakusho Co.) to obtain a sized powder. Granulation and milling granule were carried out twice, and to the resulting sized powder of Compound A (114100 g) were added croscarmellose sodium (Ac-Di-Sol) (6146 g) and magnesium stearate (1214 g) to obtain a mixed granule. The mixed granule was compressed with a tableting machine (AQUARIUS 36K, Kikusui Seisakusho Ltd.) by using a round shaped punch (9.5 mm) to prepare tablets each weighing 330 mg. To the resulting tablet (110900 g), a film coating solution consisting of hydroxypropylmethylcelluloose (3468 g), polyethylene glycol 6000 (756 g), titanium dioxide (756 g), red ferric oxide (30.24 g) and yellow ferric oxide (30.24 g) was sprayed by using a pan type coating equipment (Dria coater 1200, Powrex Corporation) so as to attain a coating of 15 mg per tablet, thereby obtaining a film-coated tablet. At this time, the product temperature was adjusted to 40°C to 50°C. Similarly, a 25 mg tablet was prepared by controlling the contents of Compound A and D-mannitol in the granules of Compound A.

**[Table 1]**

| | 25 mg tablet | 100 mg tablet |
|---|---|---|
| Compound A | 25.0 mg | 100.0 mg |
| D-mannitol | 250.0 mg | 175.0 mg |
| Sodium ascorbate | 10.0 mg | 10.0 mg |
| HPC-L | 10.0 mg | 10.0 mg |
| Ac-Di-Sol | 31.7 mg | 31.7 mg |
| Magnesium stearate | 3.3 mg | 3.3 mg |
| Hydroxypropylmethylcellulose | 10.32 mg | 10.32 mg |
| Polyethylene glycol 6000 | 2.25 mg | 2.25 mg |
| Titanium dioxide | 2.25 mg | 2.25 mg |
| Yellow ferric oxide | 0.09 mg | 0.09 mg |
| Red ferric oxide | 0.09 mg | 0.09 mg |
| Total | 345.0 mg | 345.0 mg |

### Control Example 2

A preparation was prepared according to the formulation shown in Table 2. That is, Compound A (50 g), D-mannitol (400 g), Microcrystalline cellulose (100 g), croscarmellose sodium (Ac-Di-Sol) (30 g) and sodium ascorbate (20 g) were put in a fluidized bed granulator (LAB-1, Powrex Corporation), preheated and mixed. A 6% aqueous solution of hydroxypropylcellulose (HPC-L) (333 g) was sprayed to prepare a granule of Compound A. The resulting granule of Compound A was sized by using a power mill (manufactured by Showa Kagaku Kikaikosakusho Co.) to obtain a sized powder. Granulation and milling granule were carried out twice, and to the resulting sized powder of Compound A (1085 g) were added croscarmellose sodium (Ac-Di-Sol) (58.5 g) and magnesium stearate (11.5 g) to obtain a mixed powder. The mixed powder was compressed with a tableting machine (Correct 19K, Kikusui Seisakusho Ltd.) by using a round shaped punch (9.5 mm, planar shape: two stage R (3.8-11)) to prepare tablets each weighing 330 mg.

**[Table 2]**

| | 25 mg tablet |
|---|---|
| Compound A | 25.0 mg |
| D-mannitol | 200.0 mg |
| Sodium ascorbate | 10.0 mg |
| Microcrystalline cellulose | 50.0 mg |
| HPC-L | 10.0 mg |
| Ac-Di-Sol | 31.7 mg |
| Magnesium stearate | 3.3 mg |
| Total | 330.0 mg |

### Example 1

A preparation was prepared according to the formulation shown in Table 3. To the tablet (330 g) obtained in Control Example 2, a sugar coating solution comprising erythritol (221 g), talc (68 g), gum arabic (34 g) and microcrystalline cellulose (17 g) was sprayed by using a pan type coating equipment (Hicoater 20, Freund Industrial Co., Ltd.) so as to attain a coating of 170 mg per tablet, thereby obtaining a sugar-coated tablet. At this time, the product temperature was adjusted to 35°C to 55°C.

**[Table 3]**

| | 25 mg tablet |
|---|---|
| Compound A | 25.0 mg |
| D-mannitol | 205.0 mg |
| Sodium ascorbate | 5.0 mg |
| Microcrystalline cellulose | 50.0 mg |
| HPC-L | 10.0 mg |
| Ac-Di-Sol | 31.7 mg |
| Magnesium stearate | 3.3 mg |
| Erythritol | 110.5 mg |
| Microcrystalline cellulose | 8.5 mg |
| Gum arabic | 17.0 mg |
| Sterilized Talc | 34.0 mg |
| Total | 500.0 mg |

### Example 2

A preparation was prepared according to the formulation shown in Table 4. That is, Compound A (125 g), D-mannitol (1025 g), microcrystalline cellulose (250 g), croscarmellose sodium (Ac-Di-Sol) (75 g) and sodium ascorbate (25 g) were put in a fluidized bed granulator (MP-10, Powrex Corporation), preheated and mixed. A 6% aqueous solution of hydroxypropylcellulose (HPC-L) (885 g) was sprayed to prepare a granule of Compound A. The resulting granule of Compound A was sized by using a power mill (manufactured by Showa Kagaku Kikaikosakusho Co.) to obtain a sized powder. To the resulting sized powder of Compound A (1240 g) were added croscarmellose sodium (Ac-Di-Sol) (66.8 g) and magnesium stearate (13.2 g) to obtain a mixed powder. The mixed powder was compressed with a tableting machine (Correct 19K, Kikusui Seisakusho Ltd.) by using a round shaped punch (9.5 mm, surface shape: two stage R (3.8-11)) to prepare tablets each weighing 330 mg. To the resulting tablet (330 g), a sugar coating solution comprising erythritol (221 g), talc (68 g), gum arabic (34 g) and microcrystalline cellulose (17 g) was sprayed by using a pan type coating equipment (Hicoater 20, Freund Industrial Co., Ltd.) so as to attain a coating of 170 mg per tablet, thereby obtaining a sugar-coated tablet. At this time, the product temperature was adjusted to 35°C to 55°C.

**[Table 4]**

| | 25 mg tablet |
|---|---|
| Compound A | 25.0 mg |
| D-mannitol | 200.0 mg |
| Sodium ascorbate | 10.0 mg |
| Microcrystalline cellulose | 50.0 mg |
| HPC-L | 10.0 mg |
| Ac-Di-Sol | 31.7 mg |
| Magnesium stearate | 3.3 mg |
| Erythritol | 110.5 mg |
| Microcrystalline cellulose | 8.5 mg |
| Gum arabic | 17.0 mg |
| Sterilized Talc | 34.0 mg |
| Total | 500.0 mg |

### Example 3

Compound A (500 g), D-mannitol (241.5 g), microcrystalline cellulose (95 g), light anhydrous silicic acid (3 g) and sodium ascorbate (25 g) were put in a fluidized bed granulator (MP-10, Powrex Corporation), preheated and mixed. A 6% aqueous solution of hydroxypropylcellulose (HPC-L) (475 g) was sprayed to prepare a granule of Compound A. The resulting granule of Compound A was sized by using a power mill (manufactured by Showa Kagaku Kikaikosakusho Co.) to obtain a sized powder. To the resulting sized powder of Compound A (803.7 g) were added croscarmellose sodium (Ac-Di-Sol) (42.8 g) and magnesium stearate (8.6 g) to obtain a mixed powder. The mixed powder was compressed with a tableting machine (Correct 19K, Kikusui Seisakusho Ltd.) by using a round shaped punch (8.5 mm, surface shape: sugar-coated surface (6.5 R)) to prepare tablets each weighing 190 mg.

### Example 4

A preparation was prepared according to the formulation shown in Table 5. To the tablet (190 g) obtained in Example 3, a sugar coating solution comprising erythritol (1105 g), talc (340 g), gum arabic (170 g) and Microcrystalline cellulose (85 g) was sprayed by using a pan type coating equipment (Hicoater 20, Freund Industrial Co., Ltd.) so as to attain a coating of 100 mg per tablet, thereby obtaining a sugar-coated tablet. At this time, the product temperature was adjusted to 35°C to 55°C.

### Example 5

A preparation was prepared according to the formulation shown in Table 5. To the tablet obtained in Example 3 (190 g), a sugar coating solution comprising erythritol (1105 g), talc (340 g), gum arabic (170 g) and microcrystalline cellulose (85 g) was sprayed by using a pan type coating equipment (Hicoater 20, Freund Industrial Co., Ltd.) so as to attain a coating of 170 mg per tablet, thereby obtaining a sugar-coated tablet. At this time, the product temperature was adjusted to 35°C to 55°C.

**[Table 5]**

| | Amount of sugar coating of 100 mg | Amount of sugar coating of 170 mg |
|---|---|---|
| Compound A | 100.0 mg | 100.0 mg |
| D-mannitol | 48.3 mg | 48.3 mg |
| Microcrystalline cellulose | 19.0 mg | 19.0 mg |
| Light anhydrous silicic acid | 0.6 mg | 0.6 mg |
| Sodium ascorbate | 5.0 mg | 5.0 mg |
| HPC-L | 5.7 mg | 5.7 mg |
| Ac-Di-Sol | 9.5 mg | 9.5 mg |
| Magnesium stearate | 1.9 mg | 1.9 mg |
| Erythritol | 65.0 mg | 110.5 mg |
| Microcrystalline cellulose | 5.0 mg | 8.5 mg |
| Gum arabic | 10.0 mg | 17.0 mg |
| Sterilized Talc | 20.0 mg | 34.0 mg |
| Total | 290.0 mg | 360.0 mg |

### Example 6

A preparation was prepared according to the formulation shown in Table 6. Compound A (40000 g), D-mannitol (19320 g), microcrystalline cellulose (7600 g), light anhydrous silicic acid (240 g) and sodium ascorbate (2000 g) were put in a fluidized bed granulator (WSG-60, Powrex Corporation), preheated and mixed. A 6% aqueous solution of hydroxypropylcellulose (HPC-L) (38000 g) was sprayed to prepare a granule of Compound A. The resulting granule of Compound A (67330 g) was sized by using a power mill (manufactured by Showa Kagaku Kikaikosakusho Co.) to obtain a sized powder. To the resulting sized powder of Compound A (65900 g) were added croscarmellose sodium (Ac-Di-Sol) (3506 g) and magnesium stearate (701.1 g) to obtain a mixed powder. The mixed powder was compressed with a tableting machine (AQUARIUS 36K, Kikusui Seisakusho Ltd.) by using a round shaped punch (8.5 mm) to prepare tablets each weighing 190 mg. To the resulting tablet (63650 g), a sugar coating solution comprising erythritol (21780 g), talc (6700 g), gum arabic (3350 g) and microcrystalline cellulose (1675 g) was sprayed by using a pan type coating equipment (Dria coater 1200, Powrex.Corporation). When attaining the coatings of 80 mg, 90 mg and 100 mg per tablet, a sample was taken off to obtain a sugar-coated tablet. At this time, the product temperature was adjusted to 35°C to 55°C.

**[Table 6]**

| | Amount of sugar coating of 100 mg | Amount of sugar coating of 90 mg | Amount of sugar coating of 80 mg |
|---|---|---|---|
| Compound A | 100.0 mg | 100.0 mg | 100.0 mg |
| D-mannitol | 48.3 mg | 48.3 mg | 48.3 mg |
| Microcrystalline cellulose | 19.0 mg | 19.0 mg | 19.0 mg |
| Light anhydrous silicic acid | 0.6 mg | 0.6 mg | 0.6 mg |
| Sodium ascorbate | 5.0 mg | 5.0 mg | 5.0 mg |
| HPC-L | 5.7 mg | 5.7 mg | 5.7 mg |
| Ac-Di-Sol | 9.5 mg | 9.5 mg | 9.5 mg |
| Magnesium stearate | 1.9 mg | 1.9 mg | 1.9 mg |
| Erythritol | 65.0 mg | 58.5 mg | 52.0 mg |
| Microcrystalline cellulose | 5.0 mg | 4.5 mg | 4.0 mg |
| Gum arabic | 10.0 mg | 9.0 mg | 8.0 mg |
| Sterilized Talc | 20.0 mg | 18.0 mg | 16.0 mg |
| Total | 290.0 mg | 280.0 mg | 270.0 mg |

### Example 7

A preparation was prepared according to the formulation shown in Table 7. That is, for a 100 mg tablet, Compound A (2000 g), D-mannitol (966 g), Microcrystalline cellulose (380 g), light anhydrous silicic acid (12 g) and sodium ascorbate (100 g) were put in a fluidized bed granulator (FD-5S, Powrex Corporation), preheated and mixed. A 6% aqueous solution of hydroxypropylcellulose (HPC-L) (1900 g) was sprayed to prepare a granule of Compound A. The resulting granule of Compound A (3125.5 g) was sized by using a power mill (manufactured by Showa Kagaku Kikaikosakusho Co.) to obtain a sized powder. To the resulting sized powder of Compound A (3214.8 g) were added croscarmellose sodium (Ac-Di-Sol) (166.3 g) and magnesium stearate (33.3 g) to obtain a mixed granule. The mixed granule was compressed with a tableting machine (AQUARIUS 19K, Kikusui Seisakusho Ltd.) by using a round shaped punch (8.5 mm) to prepare tablets each weighing 190 mg. To the resulting tablet (2850 g), a sugar coating solution comprising erythritol (1462.5 g), talc (450 g), gum arabic (225 g) and microcrystalline cellulose (112.5 g) was sprayed by using a pan type coating equipment (Dria coater 500, Powrex Corporation) so as to attain a coating of 100 mg per tablet, thereby obtaining a sugar-coated tablet. At this time, the product temperature was adjusted to 35°C to 55°C. To the resulting sugar-coated tablet (1160 g), a film coating solution comprising hydroxypropylmethylcellulose (111.7 g), polyethylene glycol 6000 (32 g), titanium dioxide (16 g) and yellow ferric oxide (0.32 g) was sprayed by using a pan type coating equipment (Hicoater 30, Freund Industrial Co., Ltd.) so as to attain a coating of 8 mg per tablet, thereby obtaining a film-coated tablet. At this time, the product temperature was adjusted to 40°C to 50°C. Similarly, a 25 mg tablet was prepared by controlling the contents of Compound A and D-mannitol in the granules of Compound A.

### Example 8

A preparation was prepared according to the formulation shown in Table 7. That is, for a 100 mg tablet, Compound A (40000 g), D-mannitol (19320 g), Microcrystalline cellulose (7600 g), light anhydrous silicic acid (240 g) and sodium ascorbate (2000 g) were put in a fluidized bed granulator (WSG-60, Powrex Corporation), preheated and mixed. A 6% aqueous solution of hydroxypropylcellulose (HPC-L) (38000 g) was sprayed to prepare a granule of Compound A. The resulting granule of Compound A (67330 g) was sized by using a power mill (manufactured by Showa Kagaku Kikaikosakusho Co.) to obtain a sized powder. To the resulting sized powder of Compound A (66080 g) were added croscarmellose sodium (Ac-Di-Sol) (3515 g) and magnesium stearate (703 g) to obtain a mixed powder. The mixed powder was compressed with a tableting machine (AQUARIUS 36K, Kikusui Seisakusho Ltd.) by using a round shaped punch (8.5 mm) to prepare tablets each weighing 190 mg. To the resulting tablet (66500 g), a sugar coating solution comprising erythritol (22750 g), talc (7000 g), gum arabic (3500 g) and microcrystalline cellulose (1750 g) was sprayed by using a pan type coating equipment (Dria coater 1200, Powrex Corporation) so as to attain a coating of 100 mg per tablet, thereby obtaining a sugar-coated tablet. At this time, the product temperature was adjusted to 35°C to 55°C. To the resulting sugar-coated tablet (100600 g), a film coating solution comprising hydroxypropylmethylcellulose (1938 g), polyethylene glycol 6000 (555.2 g), Titanium dioxide (277.6 g) and yellow ferric oxide (5.552 g) was sprayed by using a pan type coating equipment (Dria coater 1200, Powrex Corporation) so as to attain a coating of 8 mg per tablet, thereby obtaining a film-coated tablet. At this time, the product temperature was adjusted to 40°C to 50°C. Similarly, a 25 mg tablet was prepared by controlling the contents of Compound A and D-mannitol in the granules of Compound A.

**[Table 7]**

| | 25 mg tablet | 100 mg tablet |
|---|---|---|
| Compound A | 25.0 mg | 100.0 mg |
| D-mannitol | 123.3 mg | 48.3 mg |
| Microcrystalline cellulose | 19.0 mg | 19.0 mg |
| Light anhydrous silicic acid | 0.6 mg | 0.6 mg |
| Sodium ascorbate | 5.0 mg | 5.0 mg |
| HPC-L | 5.7 mg | 5.7 mg |
| Ac-Di-Sol | 9.5 mg | 9.5 mg |
| Magnesium stearate | 1.9 mg | 1.9 mg |
| Erythritol | 65.0 mg | 65.0 mg |
| Microcrystalline cellulose | 5.0 mg | 5.0 mg |
| Gum arabic | 10.0 mg | 10.0 mg |
| Sterilized Talc | 20.0 mg | 20.0 mg |
| Hydroxypropylmethylcellulose | 5.584 mg | 5.584 mg |
| Polyethylene glycol 6000 | 1.6 mg | 1.6 mg |
| Titanium dioxide | 0.8 mg | 0.8 mg |
| Yellow ferric oxide | 0.016 mg | 0.016 mg |
| Total | 298.0 mg | 298.0 mg |

### Example 9

A preparation was prepared according to the formulation shown in Table 8. That is, for a 25 mg tablet, Compound A (250 g), D-mannitol (2500 g), sodium ascorbate (100 g) and croscarmellose sodium (Ac-Di-Sol) (150 g) were put in a fluidized bed granulator (FD-3S, Powrex Corporation), preheated and mixed. A 5o aqueous solution of hydroxypropylcellulose (HPC-L) (2000 g) was sprayed to prepare a granule of Compound A. The resulting granule of Compound A was sized by using a power mill (manufactured by Showa Kagaku Kikaikosakusho Co.) to obtain a sized powder. Granulation and milling granule were carried out twice, and to the resulting sized powder of Compound A (2945 g) were added croscarmellose sodium (Ac-Di-Sol) (158.65 g) and magnesium stearate (31.35 g) to obtain a mixed granule. The mixed granule was compressed with a tableting machine (AQUARIUS 36K, Kikusui Seisakusho Ltd.) by using a round shaped punch (9.5 mm) to prepare tablets each weighing 330 mg. For the resulting tablet (1700 g), sugar-coating was carried out according to a conventional method by means of a three-roll sugar coating machine (16-inch pan, Kikusui Seisakusho Ltd.) using a kneading solution comprising talc (1026 g), granulated sugar (1820 g), titanium dioxide (112 g), gum arabic (172.2 g) and purified water (910 g), a dusting powder comprising talc (2631 g) and gum arabic (53.7 g) and a syrup solution comprising granulated sugar (1620 g) and purified water (809.7 g) so as to attain a coating of 300 mg per tablet, thereby obtaining a sugar-coated tablet.

**[Table 8]**

| | 25 mg tablet |
|---|---|
| Compound A | 25.0 mg |
| D-mannitol | 250.0 mg |
| Sodium ascorbate | 10.0 mg |
| HPC-L | 10.0 mg |
| Ac-Di-Sol | 16.7 mg |
| Magnesium stearate | 3.3 mg |
| Granulated sugar | 145.0 mg |
| Titanium dioxide | 5.6 mg |
| Gum arabic | 10.4 mg |
| Sterilized Talc | 139.0 mg |
| Total | 630.0 mg |

### Evaluation Example 1

Evaluation on the stability of the preparation prepared in Control Example 2 and Example 1 was performed by subdividing each preparation on a glass petri dish, keeping the subdivided preparations in a system with humidity adjusted to 33% RH at 40°C (relative humidity 33%) for 4 weeks, respectively, and measuring the residual ratio and the related substance amount (decomposition product). Table 9 shows a result of evaluation on the stability of the 25 mg tablet of Compound A. Hereinafter, the contents of Compound A and the related substances were measured by a HPLC method under the following conditions.

Content: Ten preparations were used, a precise amount of 25 mL of an internal standard solution A was added thereto, 75 mL of acetonitrile and 25 mL of a 10 mM ammonium acetate solution were further added thereto. Then, the mixture was mixed with shaking and subjected to ultrasonication. After the ultrasonication, the reaction mixture was shaken vigorously, 125 mL of acetonitrile was added thereto, and the mixture was shaken vigorously. 5 mL of this suspension was taken and the extraction solvent was added thereto, to obtain 100 mL of a solution. Determination of the prepared solution was carried out with the following measurement condition.
Internal standard solution A: solution of 2-naphthyl benzoate in acetonitrile (1 g→25 g)
Internal standard solution B: a precise amount of 25 mL of the internal standard solution A was weighted, and
acetonitrile was added thereto to obtain a precise amount of 100 mL of a solution.
Extraction Solvent: mixed solution of 10 mM ammonium acetate solution/acetonitrile (7 : 3)
Measurement wavelength: 287 nm
Column: CHIRALCEL OJ-R 5 µm 4.6 mm × 150 mm (manufactured by Daicel Chemical Industries, Ltd.)
Mobile phase: mixed solution of 10 mM ammonium acetate solution/acetonitrile (9 : 16)
Oven temperature: around 25°C

Related substance 1: Ten preparations were used, and 100 mL of acetonitrile and 25 mL of a 10 mM ammonium acetate solution were added thereto. Then, the mixture was mixed with shaking and subjected to ultrasonication. After the ultrasonication, the reaction mixture was shaken vigorously, 125 mL of acetonitrile was added thereto, and the mixture was shaken vigorously. 5 mL of this suspension was taken and the extraction solvent was added thereto, to obtain 100 mL of a solution. Determination of the prepared solution was carried out with the following measurement condition.
Extraction Solvent: mixed solution of 10 mM ammonium acetate solution/acetonitrile (7 : 3)
Measurement wavelength: 287 nm
Column: CHIRALCEL OJ-R 5 µm 4.6 mm × 150 mm (manufactured by Waters Co., Ltd.)
Mobile phase: mixed solution of 10 mM ammonium acetate solution/acetonitrile (16 : 9)
Oven temperature: around 25°C

Related substance 2: Ten preparations were used, and 100 mL of acetonitrile and 25 mL of a 10 mM ammonium acetate solution were added thereto. Then, the mixture was mixed with shaking and subjected to ultrasonication. After the ultrasonication, the reaction mixture was shaken vigorously, 125 mL of acetonitrile was added thereto, and the mixture was shaken vigorously. 5 mL of this suspension was taken and the extraction solvent was added thereto, to obtain 100 mL of a solution. Determination of the prepared solution was carried out with the following measurement condition.
Extraction solvent: mixed solution of 10 mM ammonium acetate solution/acetonitrile (3 : 4)
Measurement wavelength: 287 nm
Column: CAPCELL PAK C18 MG 5 µm 4.6 mm × 150 mm (manufactured by Shiseido Co., Ltd.)
Mobile phase: gradient of mixed solution of 10 mM ammonium acetate solution/acetonitrile (50 : 1) and mixed solution of acetonitrile/10 mM ammonium acetate solution (9 : 1)
Oven temperature: around 25°C
Related substance 3: Ten preparations were used, and 100 mL of acetonitrile and 25 mL of a 10 mM ammonium acetate solution were added thereto. Then, the mixture was mixed with shaking and subjected to ultrasonication. After the ultrasonication, the reaction mixture was shaken vigorously, 125 mL of acetonitrile was added thereto, and the mixture was shaken vigorously. 5 mL of this suspension was taken and the extraction solvent was added thereto, to obtain 100 mL of a solution. Determination of the prepared solution was carried out with the following measurement condition.
Extraction Solvent: mixed solution of 10 mM ammonium acetate solution/acetonitrile (3 : 7)
Measurement wavelength: 287 nm
Column: XTerra MS C18 3.5 µm 4.6 mm × 150 mm (manufactured by Shiseido Co., Ltd.)
Mobile phase: gradient of mixed solution of 10 mM ammonium acetate solution/acetonitrile (4 : 3) and mixed solution of acetonitrile/10 mM ammonium acetate solution (9 : 1)
Oven temperature: around 25°C

As a result, as shown in Table 9, a significant increase of the related substance amount in the core tablet of Control Example 2 was recognized. The formation of the related substances (decomposition product) was remarkably suppressed by obtaining the sugar-coated tablet obtained in Example 1, which confirms improvement in the stability.

**[Table 9]**

| | Keeping condition and Evaluation time point | Residual ratio | Total related substances |
|---|---|---|---|
| Control Example 2 (core tablet) | Initiation | 100.0% | 1.02% |
| Control Example 2 (core tablet) | 40°C, 33% RH, 1 month | 98.5% | 2.42% |
| Example 1 (sugar-coated tablet) | Initiation | 100.0% | 1.02% |
| Example 1 (sugar-coated tablet) | 40°C, 33% RH, 1 month | 98.7% | 1.16% |

### Evaluation Example 2

Evaluation on the stability of the preparation prepared in Example 2 was performed by subdividing each preparation on a glass petri dish, keeping the subdivided preparations in a system with humidity adjusted to 33% RH at 25°C (relative humidity 33%) for 2 weeks and 2 months, respectively, keeping them in a system with humidity adjusted to 33% RH at 40°C (relative humidity 33%) for 1 month, respectively and measuring the residual ratio and the related substance amount (decomposition product). Table 10 shows a result of evaluation of the stability of the preparation of the 25 mg tablet of Compound A. As a result, it was confirmed to have stability as shown in Table 10.

**[Table 10]**

| | Keeping condition and Evaluation time point | Residual ratio | Total related substances |
|---|---|---|---|
| Example 2 (sugar-coated tablet) | Initiation | 100.0% | 1.13% |
| Example 2 (sugar-coated tablet) | 25°C, 33% RH, 2 weeks | 99.8% | 1.24% |
| Example 2 (sugar-coated tablet) | 25°C, 33% RH, 2 months | 98.0% | 1.31% |
| Example 2 (sugar-coated tablet) | 40°C, 33% RH, 1 month | 100.7% | 1.25% |

### Evaluation Example 3

Evaluation on the stability of the preparation prepared in Example 4 and Example 5 was performed by subdividing each preparation in a container sealed with oxygen gas (oxygen gas concentration: 95% or more), keeping the subdivided preparations at 40°C for 2 weeks, respectively, and measuring the residual ratio and the related substance amount (decomposition product). Table 11 shows the results of evaluation on the stability of the preparation with the amounts of sugar coatings of 100 mg and 170 mg for the 100 mg tablet of Compound A. As a result, it was confirmed to have stability as shown in Table 11.

**[Table 11]**

| | Keeping condition and Evaluation time point | Residual ratio | Total related substances |
|---|---|---|---|
| Example 4 (Amount of sugar coating: 100 mg) | Initiation | 100.0% | 1.78% |
| Example 4 (Amount of sugar coating: 100 mg) | Oxygen substitution, 40°C, 2 weeks | 100.4% | 1.77% |
| Example 5 (Amount of sugar coating: 170 mg) | Initiation | 100.0% | 1.80% |
| Example 5 (Amount of sugar coating: 170 mg) | Oxygen substitution, 40°C, 2 weeks | 99.8% | 1.74% |

### Evaluation Example 4

Evaluation on the stability of the preparation, which was prepared in Example 6 and had different amounts of sugar coating (amount of coating: 80 mg, 90 mg and 100 mg), and the 100 mg preparation prepared in Control Example 1 was performed by subdividing each preparation in a container sealed with oxygen gas (oxygen gas concentration: 95% or more), keeping the subdivided preparations at 40°C for 4 weeks, respectively, and measuring the residual ratio and the related substance amount (decomposition product). Table 12 shows a result of evaluation on the stability of the preparation with the amounts of the sugar coatings of 80 mg, 90 mg and 100 mg for the 100 mg tablet of Compound A. As a result, it was confirmed to have stability as shown in Table 12. In the preparation of Control Example 1 wherein sugar coating was not performed, a remarkable increase in the related substance amount (decomposition product) was recognized.

**[Table 12]**

| | Keeping condition and Evaluation time point | Residual ratio | Total related substances |
|---|---|---|---|
| Example 6 (Amount of sugar coating: 80 mg) | Initiation | 100.0% | 1.01% |
| Example 6 (Amount of sugar coating: 80 mg) | Oxygen substitution, 40°C, 4 weeks | 98.4% | 0.98% |
| Example 6 (Amount of sugar coating: 90 mg) | Initiation | 100.0% | 1.01% |
| Example 6 (Amount of sugar coating: 90 mg) | Oxygen substitution, 40°C, 4 weeks | 96.5% | 0.98% |
| Example 6 (Amount of sugar coating: 100 mg) | Initiation | 100.0% | 1.01% |
| Example 6 (Amount of sugar coating: 100 mg) | Oxygen substitution, 40°C, 4 weeks | 98.4% | 0.98% |
| Control Example 1 (film-coated tablet) | Initiation | 100.0% | 3.30% |
| Control Example 1 (film-coated tablet) | Oxygen substitution, 40°C, 4 weeks | 91.8% | 10.16% |

### Evaluation Example 5

Evaluation on the stability of the 25 mg preparation and the 100 mg preparation of Compound A prepared in Example 7 wherein film coating was carried out on the sugar-coated layer, was performed by subdividing each preparation in a vial, tightly stoppering the vial, keeping the subdivided preparations in a system with humidity adjusted to 75% RH at 40°C (relative humidity 75%) for 1 month, respectively, and measuring the residual ratio and the related substance amount (decomposition product). Table 13 shows a result of evaluation on the stability of the preparations of the 25 mg tablet and the 100 mg tablet of Compound A. As a result, the formation of the related substances (decomposition product) was not recognized in any preparation, which confirms that they were stable as shown in Table 13.

**[Table 13]**

| | Keeping condition and Evaluation time point | Residual ratio | Total related substances |
|---|---|---|---|
| Example 7 (25 mg) | Initiation | 100.0% | 1.14% |
| Example 7 (25 mg) | 40°C, 1 month | 100.2% | 1.11% |
| Example 7 (100 mg) | Initiation | 100.0% | 1.53% |
| Example 7 (100 mg) | 40°C, 1 month | 100.9% | 1.41% |

### Evaluation Example 6

Evaluation on the stability of the 25 mg tablet of Compound A and the 100 mg tablet of Compound A prepared in Control Example 1 was performed by subdividing each preparation on a glass petri dish, keeping the subdivided preparations in a system with humidity adjusted to 33% RH at 40°C (relative humidity 33%) and a system with humidity adjusted to 75% RH at 40°C (relative humidity 75%) for 6 months, respectively, and measuring the residual ratio and the content of an oxidative decomposition product (after 1 month and after 6 months). Table 14 shows a result of evaluation on the stability of the 25 mg tablet and the 100 mg tablet of Compound A. As is clear from Table 14, an increase of the related substances was recognized.

**[Table 14]**

| | Keeping condition and Evaluation time point | Residual ratio | Total related substances |
|---|---|---|---|
| Control Example 1 (25 mg) | Initiation | 100.0% | 1.77% |
| Control Example 1 (25 mg) | 40°C, 33% RH, 1 month | 95.8% | 4.83% |
| Control Example 1 (25 mg) | 40°C, 33% RH, 6 months | 92.0 % | 8.48% |
| Control Example 1 (25 mg) | 40°C, 75% RH, 1 month | 98.5% | 2.33% |
| Control Example 1 (25 mg) | 40°C, 33% RH, 6 months | 92.0 % | 8.48% |
| Control Example 1 (100 mg) | Initiation | 100.0% | 3.21% |
| Control Example 1 (100 mg) | 40°C, 33% RH, 1 month | 97.0% | 6.13% |
| Control Example 1 (25 mg) | 40°C, 33% RH, 6 months | 92.0 % | 8.48% |
| Control Example 1 (100 mg) | 40°C, 75% RH, 1 month | 99.5% | 4.38% |
| Control Example 1 (25 mg) | 40°C, 33% RH, 6 months | 92.0 % | 8.48% |

### Evaluation Example 7

Evaluation on the stability of the 25 mg preparation and the 100 mg preparation of Compound A prepared in Example 7 wherein film coating was carried out on the sugar-coated layer, was performed by subdividing each preparation in a vial, keeping the subdivided preparations in a system into which a drying agent was put at 40°C, a system with humidity adjusted to 44% RH at 40°C (relative humidity 44%) and a system with humidity adjusted to 75% RH at 40°C (relative humidity 75%) while the vial was opened, for 10 months, respectively, and measuring the residual ratio and the related substance amount (decomposition product). As a result, the formation of the related substances (decomposition product) was not recognized in any preparation, which confirms that they were stable as shown in Table 15.

**[Table 15]**

| | Evaluation time point | Residual ratio | Total related substances |
|---|---|---|---|
| Example 7 (25 mg) | Initiation | 100.0 % | 1.46 % |
| Example 7 (25 mg) | 40°C, drying agent, 10 months | 101.9 % | 1.44 % |
| Example 7 (25 mg) | 40°C, 44% RH, 10 months | 103.1 % | 1.47 %. |
| Example 7 (25 mg) | 40°C, 75% RH, 10 months | 102.7 % | 1.48 % |
| Example 7 (100 mg) | Initiation | 100.0 % | 1.41 % |
| Example 7 (100 mg) | 40°C, drying agent, 10 months | 98.3 % | 1.41 % |
| Example 7 (100 mg) | 40°C, 44% RH, 10 months | 98.5 % | 1.38 % |
| Example 7 (100 mg) | 40°C, 75% RH, 10 months | 97.0 % | 1.38 % |

### Evaluation Example 8

Elution of each 25 mg preparation of Compound A prepared in Example 9 and Example 7 was compared. As a result, the preparation of Example 7 was eluted significantly faster compared to the preparation of Control Example 3 as shown in Fig. 1.
Elution test condition: Japanese Pharmacopoeia paddle method, 100 rpm, 37°C, Elution test solution: 0.05 mol/L citric acid buffer (pH 3.0) containing 0.3 mol/L of sodium lauryl sulfate, 900 mL

### Industrial Applicability

According to the present invention, a preparation wherein the oxidation of an active ingredient unstable to oxygen is suppressed is provided.

## Claims

1. A preparation which comprises a portion containing an active ingredient unstable to oxygen and a sugar-coated layer containing (1) sugar alcohol as a sugar-coating base material and (2) a binder, wherein the portion is coated with the sugar-coated layer.

2. The preparation according to claim 1, wherein the sugar alcohol is one or more sugar alcohols selected from erythritol, mannitol, xylitol and sorbitol.

3. The preparation according to claim 1, wherein the sugar alcohol is erythritol.

4. The preparation according to claim 1, which further contains a sugar as the sugar-coating base material.

5. The preparation according to claim 1, wherein the binder is gum arabic.

6. The preparation according to claim 1 which further comprises a film coat layer, wherein the sugar-coated layer is coated with the film coat layer.

7. The preparation according to claim 1, which further comprises an antioxidant.

8. The preparation according to claim 1, wherein the active ingredient unstable to oxygen is (R)-5,6-dimethoxy-2-[2,2,4,6,7-pentamethyl-3-(4-methylphenyl)-2,3-dihydro-1-benzofuran-5-yl]isoindoline.

9. The preparation according to claim 1, wherein the weight of the sugar-coated layer is about 20% to about 40% of the total weight of the preparation.

10. The preparation according to claim 1, wherein the sugar-coated layer contains the binder of about 1 to about 50% (w/w).

11. The preparation according to claim 1, which is a tablet.

12. A method for suppressing the oxidation of an active ingredient which comprises, in a preparation containing an active ingredient oxidizable by oxygen, coating a portion containing the active ingredient with a sugar-coated layer containing (1) sugar and/or sugar alcohol as a sugar-coating base material and (2) a binder.

13. A method for suppressing oxygen permeation of a sugar-coated layer, which comprises, in a preparation which comprises a portion containing an active ingredient oxidizable by oxygen and a sugar-coated layer, incorporating a binder into the sugar-coated layer.

14. A method for producing a sugar-coated preparation wherein the oxidation of an active ingredient is suppressed, which comprises coating a portion containing the active ingredient oxidizable by oxygen with a sugar-coated layer containing (1) sugar and/or sugar alcohol as a sugar-coating base material and (2) a binder.
